# EUROPÄISCHE PATENTSCHRIFT

(11) **EP 2 422 710 B1**
(45) Veröffentlichungstag und Bekanntmachung des Hinweises auf die Patenterteilung: **23.04.2014**
(21) Anmeldenummer: 11006844.2
(22) Anmeldetag: 22.08.2011
(51) Int. Cl.: A61B 17/02

(54) **System zum distrahieren eines Bandscheibenfachs**
System for distracting an intervertebral disc space
Système de distraction d'un espace intervertébral

(30) Priorität: 24.08.2010 DE 102010035266
(43) Veröffentlichungstag der Anmeldung: 29.02.2012
(73) Patentinhaber: spontech spine intelligence group AG, 70182 Stuttgart (DE)
(72) Erfinder: Copf, Franz, 70184 Stuttgart (DE); Mohr, Marcus, 70839 Gerlingen (DE); Taddia, Lino, 78333 Stockach (DE); Fischer, Erik, 70619 Stuttgart (DE); Anic, Tomislav, 70599 Stuttgart (DE); Pross, Gerhard, 71039 Weil im Schönbuch (DE)
(74) Vertreter: Schwanhäußer, Gernot

(56) Entgegenhaltungen:
- EP-A2- 0 796 593
- WO-A1-2004/024005
- WO-A1-2006/060430
- DE-C2- 10 003 050

## Beschreibung

### HINTERGRUND DER ERFINDUNG

### 1. Gebiet der Erfindung

Die Erfindung betrifft ein System zum Distrahieren eines Bandscheibenfachs, wie es aus der DE 100 03 050 C2 bekannt ist.

### 2. Beschreibung des Standes der Technik

Bei degenerierten Wirbelsäulensegmenten besteht eine Behandlungsmöglichkeit darin, die Bandscheibe ganz oder teilweise zu entfernen und ein Implantat in das Bandscheibenfach einzusetzen. Häufig ist das Implantat als eine Art Schraube oder Käfig ausgebildet und wird in das Bandscheibenfach hineingeschraubt oder geschlagen. Das Implantat hält die benachbarten Wirbel auf Abstand und ermöglicht es, dass das Bandscheibenfach nach und nach verknöchert, was schließlich zu einer Fusion der benachbarten Wirbel führt. Daneben gibt es auch bewegliche Implantate, die meist als Prothesen bezeichnet werden und die Beweglichkeit der benachbarten Wirbel erhalten.

Degenerierte Bandscheiben sind häufig so flach, dass der Abstand zwischen den das Bandscheibenfach begrenzenden Wirbeln vergrößert werden muss. Das Gleiche gilt, wenn eine Bandscheibe entfernt wird. Zum Distrahieren des Bandscheibenfachs werden Distraktoren eingesetzt, die in das Bandscheibenfach eingeführt werden.

Werden Implantate minimalinvasiv in das Bandscheibenfach eingesetzt, so steht nur eine sehr kleine Zugangsöffnung zum Bandscheibenfach zur Verfügung. Da andererseits zum Distrahieren von Wirbeln relativ große Kräfte benötigt werden, scheiden feinmechanische Lösungen aus.

Zum Distrahieren eines Bandscheibenfachs schlägt die oben genannte DE 100 03 050 C2 ein System vor, das mehrere Distraktoren aufweist. Jeder Distraktor ist mit einem Distraktionskopf versehen, der im Querschnitt die Form eines Ovals hat. Durch Drehen des Distraktors um seine Längsachse wird der Distraktionskopf im Bandscheibenfach aufgerichtet, wodurch dieses distrahiert wird. Um die Distraktion des Bandscheibenfachs zu erhalten und den Distraktor entfernen zu können, wird eine Hülse auf den Distraktor aufgeschoben, die an ihrem distalen Ende zwei Distanzzungen hat, die in das Bandscheibenfach hineinreichen. Die Breite der Distanzzungen ist dabei so bemessen, dass sie das Bandscheibenfach in der distrahierten Stellung halten. Dadurch kann nach dem Entfernen des Distraktors ein Implantat oder zuvor ein Bohrer in das Bandscheibenfach eingesetzt werden. Falls eine weitere Distraktion des Bandscheibenfachs gewünscht und möglich ist, so wird neben die Hülse ein weiterer Distraktor eingeführt, dessen Distraktionskopf größere Abmessungen hat. Durch Drehen des Distraktors wird dessen Distraktionskopf im Bandscheibenfach aufgerichtet, wodurch dieses weiter distrahiert wird. Anschließend wird eine Hülse mit breiteren Distanzzungen über den zweiten Distraktor geschoben. Dieser Vorgang kann, eine entsprechende Distrahierbarkeit des Bandscheibenfachs vorausgesetzt, solange fortgesetzt werden, wie nebeneinander Hülsen mit unterschiedlich breiten Distanzzungen im Bandscheibenfach angeordnet werden können.

Ein Problem bei dem bekannten System besteht darin, dass die Distraktoren und Hülsen relativ lang sein müssen, damit sie mit einer ausreichenden Länge über den am Patienten freigelegten Zugangskanal hinausragen. Wird nun eine Hülse über das aus dem Zugangskanal weit herausragende proximale Ende eines Distraktors aufgeschoben, so wird die gesamte Anordnung etwas unhandlich, da dann auch das proximale Ende der Hülse sehr weit nach oben reicht. In dieser Höhe können sich beispielsweise ein Operationsmikroskop, eine Lichtquelle, ein Bildschirm o. ä. befinden. Solche Gegenstände müssen dann gegebenenfalls weggeschwenkt oder auf sonstige weise von der Operationsstelle entfernt werden, um das Aufschieben der Hülse auf den Distraktor zu ermöglichen.

Problematisch ist ferner, dass ein am Distraktor befestigter Griff entfernt werden muss, bevor die Hülse über das proximale Ende des Distraktors aufgeschoben werden kann. Ohne Griff lässt sich der Distraktor jedoch nicht mehr präzise vom Chirurgen führen. Dies kann im ungünstigen Falle die Folge haben, dass sich der Distraktionskopf des Distraktors im Bandscheibenfach unter dem Einfluss der auf ihn wirkenden Kräfte verdreht und dadurch die vorher eingestellte Distraktion ganz oder teilweise verloren geht.

### ZUSAMMENFASSUNG DER ERFINDUNG

Aufgabe der Erfindung ist es, ein System der eingangs genannten Art dahingehend zu verbessern, dass seine Handhabbarkeit erleichtert wird.

Gelöst wird diese Aufgabe durch ein System mit den im Anspruch 1 angegebenen Merkmalen. Erfindungsgemäß ist jede Hülse mit einem sich über die gesamte Länge erstreckenden Längsschlitz versehen, so dass sie seitlich auf einen der Distraktoren aufsetzbar ist.

Anstatt also die Hülse in axialer Richtung auf das proximale Ende des Distraktors aufzuschieben, wird erfindungsgemäß die Hülse seitlich auf den Distraktor, oder genauer gesagt auf dessen aus dem Zugangskanal herausragenden Abschnitt, aufgesetzt. Dadurch entfällt die Notwendigkeit, über dem proximalen Ende des Distraktors einen großen Freiraum zu schaffen, der bei dem bekannten System zum axialen Aufschieben der Hülse auf den Distraktor benötigt wird.

Vor allem jedoch muss beim seitlichen Aufsetzen der Hülse ein am Distraktor befestigter Griff nicht demontiert werden. Der Chirurg kann somit die Hülse aufsetzen und gleichzeitig den Distraktor ohne Unterbrechung an dem daran befestigten Griff halten. Die zuvor vom Chirurgen gewählte räumliche Ausrichtung des Distraktors und auch seine axiale Drehstellung gehen deswegen beim Aufsetzen der Hülse nicht verloren.

Bei dem erfindungsgemäßen System hat jeder Distraktor ein zumindest im Wesentlichen stabförmiges Hauptteil, an dessen distalem Ende ein Distraktionskopf angeordnet ist, der im Querschnitt unterschiedliche Abmessungen in zwei orthogonalen Richtungen hat. Wenn der Querschnitt die Form eines Ovals hat, so können keine scharfe Kanten empfindliches Gewebe verletzen. Unter einem Oval wird dabei ganz allgemein eine geschlossene Kurve verstanden, die glatt ist und unterschiedliche Abmessungen in zwei orthogonalen Richtungen hat. Die Glattheit lässt sich mathematisch dadurch definieren, dass die Kurve einmal stetig differenzierbar ist. Der Querschnitt kann somit auch gerade Abschnitte aufweisen, die jedoch kontinuierlich, d. h. ohne Ecken zu bilden, in die gekrümmten Abschnitte übergehen. Die Ellipse ist ein Sonderfall eines Ovals. Das Verhältnis der Halbachsen des Ovals kann dabei bei allen Distraktoren gleich sein.

Bei einem Ausführungsbeispiel ist an dem Hauptteil der Distraktoren ein Bereich mit vergrößertem Querschnitt vorgesehen, der zu einem fensterartig erweiterten Bereich im Längsschlitz der Hülsen korrespondiert. Auf diese Weise ist jede Hülse nur in einer einzigen axialen Relativposition oder, falls mehrere fensterartig erweiterte Bereiche vorgesehen sind, in mehreren axialen Relativpositionen seitlich auf einen der Distraktoren aufsetzbar.

Die Hülsen und Distraktoren können so dimensioniert sein, dass jeweils zwei unterschiedliche Distraktoren von ein und derselben Hülse aufgenommen werden können, und dass umgekehrt jeder Distraktor zumindest in zwei unterschiedlich große Hülsen aufgenommen werden kann. Auf diese Weise kann das Bandscheibenfach stufenweise distrahiert werden, ohne dass mehrere Distraktoren nebeneinander in das Eandscheibenfach eingeführt, werden müssen. Sch-agwortartig lässt sich der erfindungsgemäße Distraktionsvorgang somit wie folgt zusammenfassen: "kleiner Distraktor - kleine Hülse - größerer Distraktor - größere Hülse - noch größerer Distraktor - noch größere Hülse usw.".

Das Hauptteil des Distraktors kann einen insgesamt kreisförmigen Querschnitt haben. Wenn der Bereich mit vergrößertem Querschnitt durch eine ringförmige Verdickung gebildet wird, so kann diese zur axialen Führung des Distraktors in der Hülse dienen.

Das System kann einen Griff umfassen, der lösbar an unterschiedlichen Distraktoren befestigbar ist. Dies verringert nicht nur Herstellungskosten, sondern auch die Kosten für die Menge des beim Betrieb aufzubereitenden Materials. In diesem Fall kann der Bereich des Distraktors mit vergrößertem Querschnitt auch durch einen Befestigungsabschnitt gebildet sein, der an den Distraktoren zum lösbaren Befestigen des Griffs ausgebildet ist.

Der Griff kann in befestigtem Zustand radial von dem stabförmigen Hauptteil abstehen, so dass der Distraktor durch Verschwenken des Griffs um die Längsachse des Distraktors in der Hülse verdrehbar ist.

Das System kann ferner Axialbegrenzer aufweisen, welche die axiale Beweglichkeit der Distraktoren in den Hülsen zumindest in einer Drehstellung der Distraktoren in distaler Richtung begrenzen. Insbesondere können die Axialbegrenzer so ausgebildet sein, dass der Distraktionskopf der Distraktoren zumindest in einer Drehstellung der Distraktoren nicht über das distale Ende der jeweiligen Hülse hinausgeführt werden kann. Auf diese weise wird verhindert, dass empfindliches Gewebe an der dem Chirurgen abgewandten Seite des Bandscheibenfachs von einem zu weit eingeführten Distraktor beschädigt wird.

Der Axialbegrenzer kann eine Nase, die seitlich vom stabförmigen Hauptteil der Distraktoren absteht, und ein an den Hülsen ausgebildete und mit der Nase zusammenwirkende axiale Anschlagsfläche umfassen. Die axiale Anschlagsfläche kann sich dabei in einer Ebene erstrecken, die zumindest im Wesentlichen senkrecht zur Längsrichtung der Hülsen verläuft.

Ferner können Drehbegrenzer vorgesehen sein, welche die Beweglichkeit der Distraktoren in den Hülsen in Drehrichtung zumindest dann begrenzen, wenn sich die Distraktoren in den jeweiligen Hülsen in den durch die Axialbegrenzer vorgegebenen axialen Endpositionen befinden. Die Drehbegrenzer verhindern, dass die Distraktoren in die falsche Richtung gedreht werden und der Distraktor dann nicht mehr so zur Hülse orientiert ist, dass die Hülse seitlich vom Distraktor abgezogen werden kann.

Jeder Drehbegrenzer kann dabei die Nase, die auch für die axiale Begrenzung verwendet wird, und eine mit der Nase zusammenwirkende Drehanschlagsfläche umfassen. Die Drehanschlagsfläche erstreckt sich dabei vorzugsweise in einer Ebene, die zumindest im Wesentlichen parallel zur Längsrichtung der Hülsen verläuft.

Die axiale Anschlagsfläche und die Drehanschlagsfläche können durch Ausnehmungen definiert sein, die in zwei diametral von der Hülse abgehenden Vorsprüngen gebildet sind.

Jede Hülse kann am proximalen Ende eine radial vorspringende Einwirkfläche haben, die sich in einer Ebene senkrecht zur Längsrichtung der Hülse erstreckt. Diese Einwirkfläche kann insbesondere so ausgelegt sein, dass mit einem Hammer oder einem anderen Schlagwerkzeug die Distanzzungen vorsichtig in das Bandscheibenfach eingeschlagen werden können. Die Einwirkflächen können dabei beispielsweise an den Vorsprüngen ausgebildet sein, deren Ausnehmungen die axiale Anschlagsfläche und die Drehanschlagsfläche definieren.

Die Distanzzungen der Hülsen sind vorzugsweise Teile einer Hülsenwand und durch zwei Aussparungen in der Hülsenwand definiert, die sich am distalen Ende der Hülse einander gegenüberliegen. Die umfangsmäßige Erstreckung der Aussparungen legt somit die Breite der Distanzzungen fest.

Zwischen den Distanzzungen können die Hülsen Auflageschultern haben, deren Außenabstand voneinander größer ist als die Breite der Distanzzungen. Mit diesen Auflageschultern können die Hülsen an den das Bandscheibenfach begrenzenden Wirbeln anschlagen, wenn die Distanzzungen in das Bandscheibenfach eindringen.

Gegenstand der vorliegenden Erfindung ist ferner ein System zum Distrahieren eines Bandscheibenfachs, das mehrere Hülsen aufweist, die jeweils an ihrem distalen Ende zwei Distanzzungen haben. Die Distanzzungen haben senkrecht zur Längsrichtung der Hülse eine Breite, in der sich die Hülsen voneinander unterscheiden. Ferner weist das System mehrere Distraktoren auf, die jeweils ein zumindest im Wesentlichen stabförmiges Hauptteil haben. An dessen distalem Ende ist jeweils ein Distraktionskopf angeordnet, der im Querschnitt unterschiedliche Abmessungen in zwei orthogonalen Richtungen und insbesondere die Form eines Ovals hat. Jeder Distraktor ist in mindestens eine Hülse einführbar und darin um seine Längsachse verdrehbar.

Ein solches System kann insbesondere die Merkmale des Anspruchs 4 oder eines der Ansprüche 6 bis 15 aufweisen.

### KURZE BESCHREIBUNG DER ZEICHNUNGEN

Weitere Merkmale und Vorteile der Erfindung ergeben sich aus der nachfolgenden Beschreibung eines Ausführungsbeispiels anhand der Zeichnungen. Darin zeigen:
- Figur 1: eine perspektivische Ansicht einer Hülse, die Teil des erfindungsgemäßen Systems ist;
- Figur 2: eine Vorderansicht der in der Figur 1 gezeigten Hülse;
- Figur 3: eine Seitenansicht der in der Figur 1 gezeigten Hülse;
- Figur 4: eine Untersicht der in der Figur 1 gezeigten Hülse;
- Figur 5: eine perspektivische Ansicht eines Distraktors mit daran angebrachtem Griff, der Teil des erfindungs-gemäßen Systems ist;
- Figur 6: einen schematischen Schnitt durch ein Bandscheiben-fach, wobei sich der in der Figur 5 gezeigte Distraktor in einer ersten Drehstellung befindet;
- Figur 7: einen schematischen Schnitt durch das Bandscheiben-fach, wobei sich der Distraktor nach Drehung um 90° in einer zweiten Drehstellung befindet;
- Figur 8: eine perspektivische Ansicht des in der Figur 5 gezeigten und mit einem Griff versehenen Distrak- tors, auf den seitlich die in den Figuren 1 bis 4 gezeigte Hülse aufgesetzt ist;
- Figur 9: eine perspektivische Ansicht wie Figur 8, jedoch mit herabgeschobener Hülse;
- Figur 10: eine perspektivische Ansicht wie Figur 9, wobei die Hülse ihre axiale Endstellung erreicht hat;
- Figur 11: einen schematischen Schnitt durch das Bandscheiben-fach gemäß der Figur 5, jedoch mit zusätzlich ein-geführten Distanzzungen der Hülse;
- Figur 12: eine perspektivische Ansicht wie Figur 10, jedoch nach erneuter Drehung des Distraktors um ±90°;
- Figur 13: einen schematischen Schnitt durch das Bandscheiben-fach nach dieser erneuten Drehung des Distraktors;
- Figur 14: eine perspektivische Ansicht wie Figur 12, jedoch nach teilweisem Herausziehen des Distraktors;
- Figur 15: einen schematischen Schnitt durch das Bandscheiben-fach nach dem Herausziehen des Distraktors;
- Figur 16: eine perspektivische Ansicht wie Figur 14, jedoch nach teilweisem Einführen eines anderen Distraktors mit größerem Distraktionskopf;
- Figur 17: einen schematischen Schnitt durch das Bandscheiben-fach nach dem Einführen des anderen Distraktors;
- Figur 18: einen schematischen Schnitt durch das Bandscheiben-fach nach dem Verdrehen des anderen Distraktors;
- Figur 19: einen schematischen Schnitt durch das Bandscheiben-fach nach dem Herausziehen der Hülse;
- Figur 20: einen schematischen Schnitt durch das Bandscheiben-fach nach dem Einführen einer Hülse mit breiteren Distanzzungen.

### BESCHREIBUNG BEVORZUGTER AUSFÜHRUNGSBEISPIELE

### I. Aufbau

Das erfindungsgemäße System zum Distrahieren eines Bandscheibenfachs umfasst mehrere Hülsen und Distraktoren, die sich nur durch bestimmte Abmessungen voneinander unterscheiden.

### a) Hülse

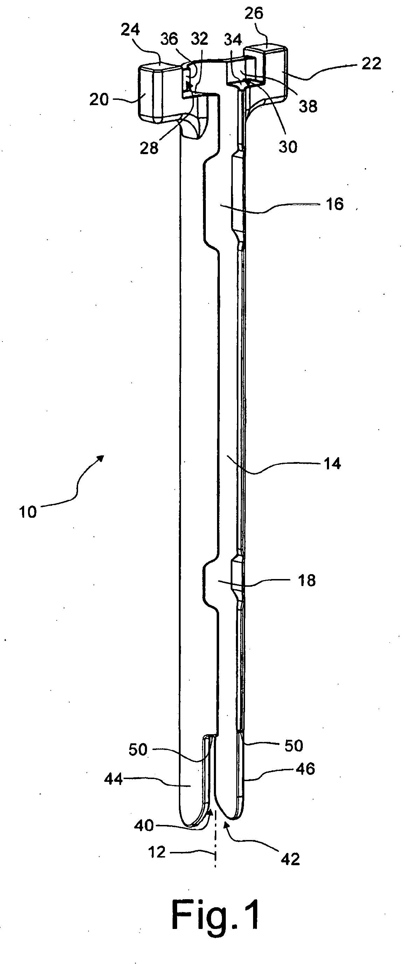

Im Folgenden wird zunächst eine der Hülsen mit Bezug auf die Figuren 1 bis 4 näher beschrieben. Diese Figuren zeigen die Hülse in einer perspektivischen Ansicht, einer Vorderansicht, einer Seitenansicht bzw. einer Untersicht.

Die insgesamt mit 10 bezeichnete Hülse hat ein proximales Ende, das in den Figuren nach oben weist und bei der Verwendung der Hülse 10 während einer Bandscheibenoperation dem Chirurgen zugewandt ist. Das gegenüberliegende, in den Figuren 1 bis 3 nach unten weisende Ende der Hülse 10 wird als distales Ende bezeichnet. Mit dem distalen Ende voran wird die Hülse 10 während der Operation durch eine vorab freigelegte Zugangsöffnung in den Patienten eingeführt.

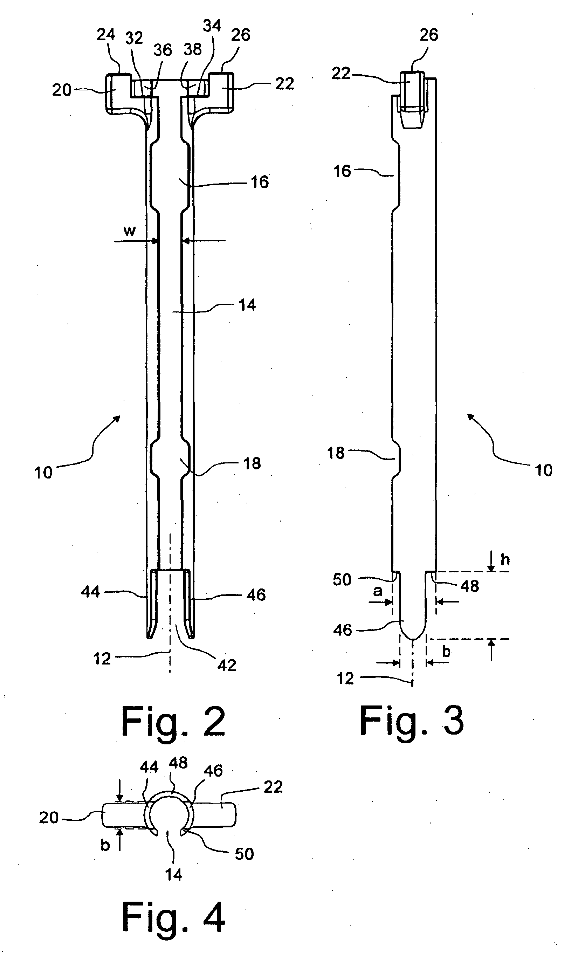

Die Hülse 10 hat im Wesentlichen einen kreisringförmigen Querschnitt; möglich sind jedoch auch andere, z.B. quadratische, Querschnitte. Parallel zu einer Längsachse 12 der Hülse 10 erstreckt sich ein Längsschlitz 14, und zwar durchgehend vom proximalen Ende bis zum distalen Ende der Hülse 10. Der Längsschlitz 14 hat eine Breite w (vgl. Figur 2), die sich nur im Bereich eines großen Fensters 16, eines kleinen Fensters 18 und am distalen Ende vergrößert, wie dies weiter unten noch näher erläutert werden wird.

Am proximalen Ende der Hülse 10 sind zwei Vorsprünge 20, 22 ausgebildet, die sich diametral einander gegenüberliegen und in radialer Richtung von der Längsachse 12 der Hülse 10 abstehen. Die proximalen Endflächen der beiden Vorsprünge 20, 22 bilden Einwirkflächen 24, 26, die eben sind und senkrecht zur Längsachse 12 der Hülse 10 verlaufen. Wie später näher erläutert werden wird, können die Einwirkflächen 24, 26 dazu verwendet werden, mit einem Hammer oder einem anderen Einschlaginstrument das distale Ende der Hülse 10 in ein Bandscheibenfach eines Patienten zu treiben.

Die beiden Vorsprünge 20, 22 sind zur Längsachse 12 hin jeweils mit einer Ausnehmung 28, 30 versehen. Die radialen Begrenzungsflächen der Ausnehmungen 28, 30 sind dabei jeweils konkav und konzentrisch zueinander gekrümmt. Bodenflächen der Ausnehmungen 28, 30, die sich in einer Ebene senkrecht zu Längsachse 12 der Hülse 10 erstrecken, bilden dabei axiale Anschlagsflächen 34, und zwei seitliche Wände, die sich in einer Ebene parallel zur Längsachse 12 erstrecken, Drehanschlagsflächen 36, 38.

An ihrem distalen Ende weist die Hülse 10 zwei diametral einander gegenüberliegende Aussparungen 40, 42 auf, zwischen denen zwei Distanzzungen 44, 46 verbleiben, die einander gegenüberliegen. Die Distanzzungen 44, 46 werden somit durch einen Abschnitt der Hülsenwand gebildet, sind aber zum distalen Ende hin annähernd elliptisch abgerundet, wie man dies am besten in der Seitenansicht der Figur 3 erkennen kann.

Die proximale Stirnfläche der Aussparung 40 bildet eine erste Auflageschulter 48, während die gegenüberliegende Aussparung 42 infolge des dort verlaufenden Längsschlitzes 14 zwei schmalere zweite Auflageschultern 50 festlegt. Der Außenabstand zwischen der Auflageschulter 48 einerseits und den Auflageschultern 50 andererseits beträgt a und ist größer als die Breite b der Distanzzungen 44, 46.

Für die Erläuterung der für die Funktion wichtigen Abmessungen der Distanzzungen 44, 46 wird im Folgenden Bezug auf die Figuren 3 und 4 genommen. Entlang einer Transversalrichtung, die senkrecht zur Längsachse 12 verläuft, haben die beiden Distanzzungen 44, 46 eine Breite b und entlang der Längsrichtung 12 eine Höhe h. Die Hülsen des erfindungsgemäßen Systems unterscheiden sich voneinander durch die Breiten b der Distanzzungen 44, 46, aber auch durch den Innen- und Außendurchmesser der Hülsen 10 und damit des Außenabstandes zwischen den Auflageschultern 48, 50.

### b) Distraktor

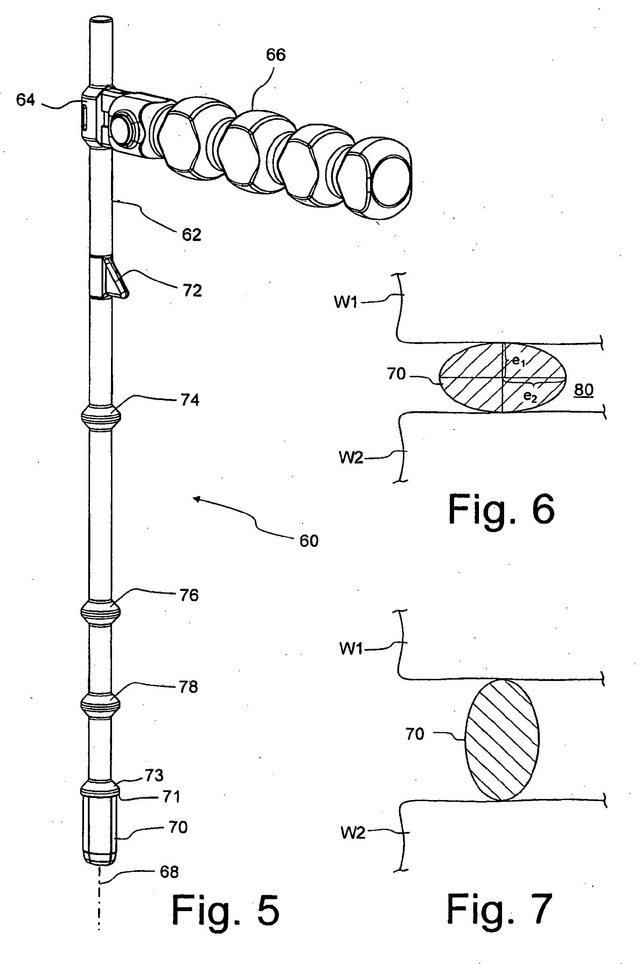

Im Folgenden wird mit Bezug auf die Figur 5 der Aufbau eines der Distraktoren erläutert. Der perspektivisch dargestellte und insgesamt mit 60 bezeichnete Distraktor hat ebenfalls ein in der Figur 5 nach oben weisendes proximales und ein in der Figur 5 nach unten weisendes distales Ende. Der Distraktor 60 besteht im Wesentlichen aus einem stabförmigen Hauptteil 62, das einen kreisförmigen Querschnitt hat. In der Nähe des proximalen Endes ist an dem Hauptteil 62 ein Befestigungsabschnitt 64 ausgebildet, der zur lösbaren Befestigung eines Drehgriffs 66 dient. Wenn der Distraktor 60 in eine Hülse 10 eingeführt ist, lässt er sich dort mit Hilfe des Drehgriffs 66 um seine Längsachse 68 drehen.

Am gegenüberliegenden distalen Ende trägt das Hauptteil 62 einen Distraktionskopf 70, der, wie die schematische Schnittdarstellung der Figur 6 zeigt, einen ovalen Querschnitt hat. Die kürzere Halbachse des Ovals ist dabei mit e₁ und die längere Halbachse mit e₂ bezeichnet. Zum distalen Ende hin ist der Distraktionskopf 70 abgerundet, so dass er keinerlei scharfe Kanten hat. Zum proximalen Ende hin wird der Distraktionskopf 70 durch eine radial vorspringende Schulter 71 begrenzt, die durch einen umlaufenden Kranz 73 gebildet wird.

In axialer Richtung hat der Distraktionskopf 70 etwa die gleiche Höhe wie die Höhe h der Distanzzungen 44, 46.

Zwischen dem Befestigungsabschnitt 64 und dem Distraktionskopf 70 sind an dem Hauptteil 62 noch eine Nase 72, welche ungefähr die Form eines rechtwinkligen Dreiecks hat, und drei ringförmige Verdickungen 74, 76, 78 ausgebildet.

Die Distraktoren 60 des erfindungsgemäßen Systems unterscheiden sich insbesondere durch den Querschnitt des Distraktionskopfs 70, d. h. die Längen der Halbachsen e₁, e₂. Außerdem variiert bei dem dargestellten Ausführungsbeispiel auch der Durchmesser des stabförmigen Hauptteils 62 und entsprechend auch der Verdickungen 74, 76, 78. Der Befestigungsabschnitt 64 ist bei allen Distraktoren 60 hingegen identisch ausgebildet. Dadurch kann ein und derselbe Drehgriff 66 an allen unterschiedlichen Distraktoren 60 befestigt werden.

### II. Verwendung

Im Folgenden wird mit Bezug auf die Figuren 6 bis 20 beschrieben, wie das vorstehend beschriebene System aus Hülsen 10 und Distraktoren 60 verwendet wird, um ein Bandscheibenfach zu distrahieren.

In einem ersten Schritt wird ein Zugang zu dem Bandscheibenfach freigelegt. Dabei kann es sich beispielsweise um einen posterior-lateralen Zugang handeln. Im den meisten Fällen wird dann die Bandscheibe ganz oder teilweise entfernt. Die das Bandscheibenfach begrenzenden Wirbel W1, W2 bewegen sich dabei im allgemeinem aufeinander zu, wodurch sich die Höhe des Bandscheibenfachs verringert.

In der Figur 6 ist etwas weniger als eine Hälfte eines so präparierten und mit 80 bezeichneten Bandscheibenfachs zwischen zwei aneinander angrenzenden Wirbeln W1, W2 gezeigt. Rechts davon schließt sich der Spinalkanal und die andere Hälfte des Bandscheibenfachs an (nicht dargestellt).

In einem zweiten Schritt wird in das so freigelegte und präparierte Bandscheibenfach 80 der Distraktionskopf 70 eines Distraktors 60 eingeführt, der so bemessen ist, dass er in der in der Figur 6 gezeigten Drehstellung gerade noch in das Bandscheibenfach 80 hineinpasst. In dieser Drehstellung verläuft die kurze Halbachse e₁ parallel zur Längsrichtung der Wirbelsäule, so dass in dieser Richtung der Distraktionskopf 70 seine minimalen Abmessungen hat. Der Distraktor 60 wird hierzu, vorzugsweise mit daran befestigtem Drehgriff, mit seinem distalen Ende voran in den freigelegten Zugangskanal eingeführt und mit Hilfe des Drehgriffs 66 in die vorstehend erwähnte Drehposition gebracht. Die oberhalb des Distraktionskopfes 70 radial vorspringende Schulter 71 schlägt schließlich an den Wirbeln W1, W2 an und verhindert so, dass das distale Ende des Distraktionskopfes 70 zu weit in das Bandscheibenfach 80 eingeführt und dadurch empfindliches Gewebe am hinteren Ende des Bandscheibenfachs 80 beschädigt wird.

In einem dritten Schritt wird der Distraktor 60 mit Hilfe des Drehgriffs 66 um etwa 90° verdreht. Der Distraktionskopf 70 im Bandscheibenfach 80 vollzieht diese Drehung mit und richtet sich dadurch auf, wie dies in der Figur 7 erkennbar ist. Während der Drehung spreizt der Distraktionskopf 70 das Bandscheibenfach 80 nach und nach auf, bis der Abstand zwischen den Wirbeln W1, W2 gleich der doppelte Länge der langen Halbachse e₂ ist. Durch diese Drehung wurde das Bandscheibenfach 80 somit um den Betrag 2 (e₂-e₁) distrahiert. Dieser Zustand ist in der Figur 7 gezeigt.

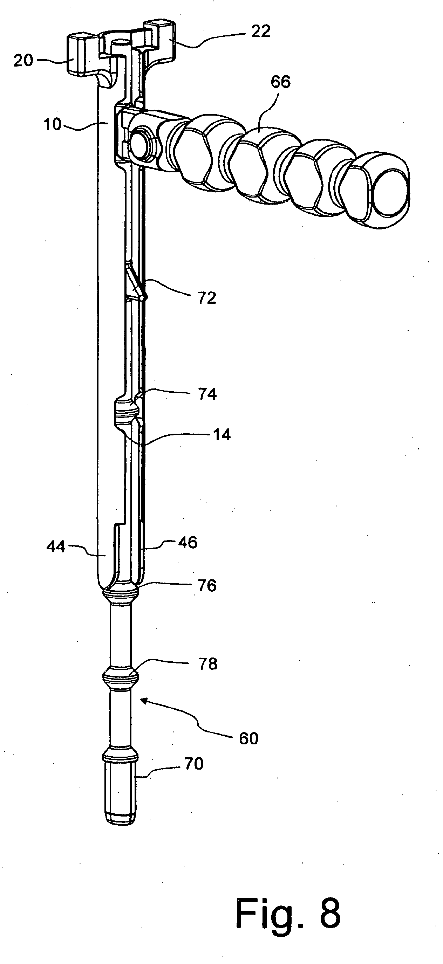

In einem vierten Schritt wird eine Hülse 10 seitlich auf den Distraktor 60 aufgesetzt, wie dies in der perspektivischen Darstellung der Figur 8 erkennbar ist. Verwendet wird dabei aus dem Satz der zur Verfügung stehenden Hülsen diejenige, bei der die Breite b der Distanzzungen 44, 46 zumindest annähernd gleich der jetzt eingestellten Höhe 2·e₂ des Bandscheibenfachs 80 beträgt.

Wie man in der Figur 8 erkennt, nimmt beim seitlichen Aufsetzen der Hülse 10 deren großes Fenster 16 den Befestigungsabschnitt 64 des Distraktors 60 auf. Entsprechendes gilt für das kleine Fenster 18 der Hülse 10 und die obere ringförmige Verdickung 74 am Distraktor 60. Für die beiden darunter angeordneten ringförmigen Verdickungen 76, 78 des Distraktors 60 sind keine Fenster erforderlich, da sie sich beim Aufsetzen der Hülse 10 auf den Distraktor 60 unterhalb des distalen Endes der Hülse 10 befinden. Aus der Figur 8 wird ferner deutlich, dass die Hülse 10 nur in der dargestellten Axialposition seitlich auf den Distraktor 60 aufgesetzt werden kann. In anderen relativen Axialpositionen verhindern der Befestigungsabschnitt 64 und die Verdickungen 74, 76, 78 ein seitliches Aufsetzen.

Beim Aufsetzen der Hülse 10 auf den Distraktor 60 kann der Griff 66 somit am Distraktor 60 befestigt bleiben. Der Chirurg erhält dadurch die Möglichkeit, die räumliche Ausrichtung des Distraktors 60 und auch dessen axiale Drehstellung während des Aufsetzens der Hülse 10 unverändert zu lassen. Ferner kann ein Operationsmikroskop, das beim Einführen des Distraktors 60 im zweiten Schritt zur Kontrolle verwendet wurde, an Ort und Stelle verbleiben und muss nicht weggeschwenkt werden. Dies ist deswegen von Bedeutung, weil im Allgemeinen auch im nachfolgenden fünften Schritt das Operationsmikroskop zur Kontrolle benötigt wird.

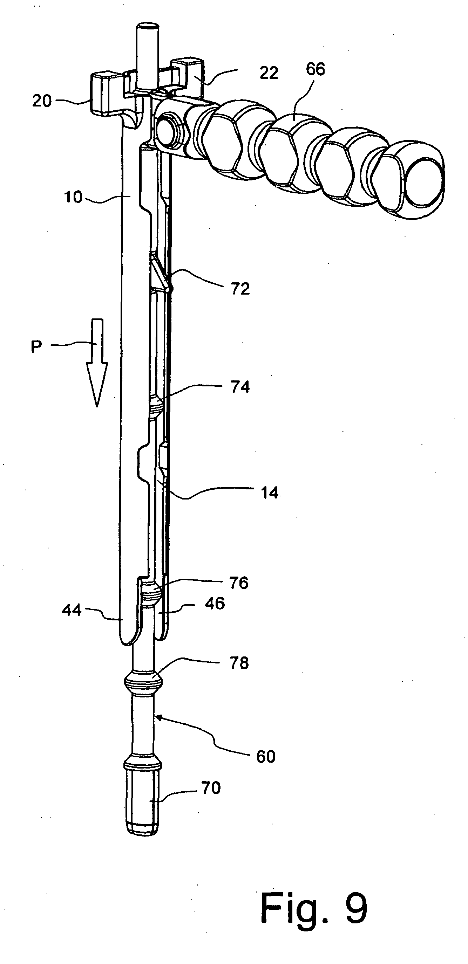

In diesem fünften Schritt wird die seitlich aufgesetzte Hülse 10 in axialer Richtung am Distraktor 60 entlang abgesenkt, wie dies in der perspektivischen Darstellung der Figur 9 erkennbar und durch einen Pfeil P angedeutet ist. Die Verdickungen 74, 76, 78 dienen dabei als radiale Führungen, die den Distraktor 60 axialbeweglich in der Hülse 10 zentrieren, und stellen so sicher, dass der Distraktor 60 nicht in der Hülse 10 verkippt und die Distanzzungen 44, 46 an dem umlaufenden Kranz 73 des Distraktors 60 aufsitzen. Während das in der Figur 8 gezeigte seitliche Aufsetzen noch außerhalb des Patienten durchgeführt wird, dringt die Hülse 10 beim axialen Absenken in die Zugangsöffnung des Patienten ein.

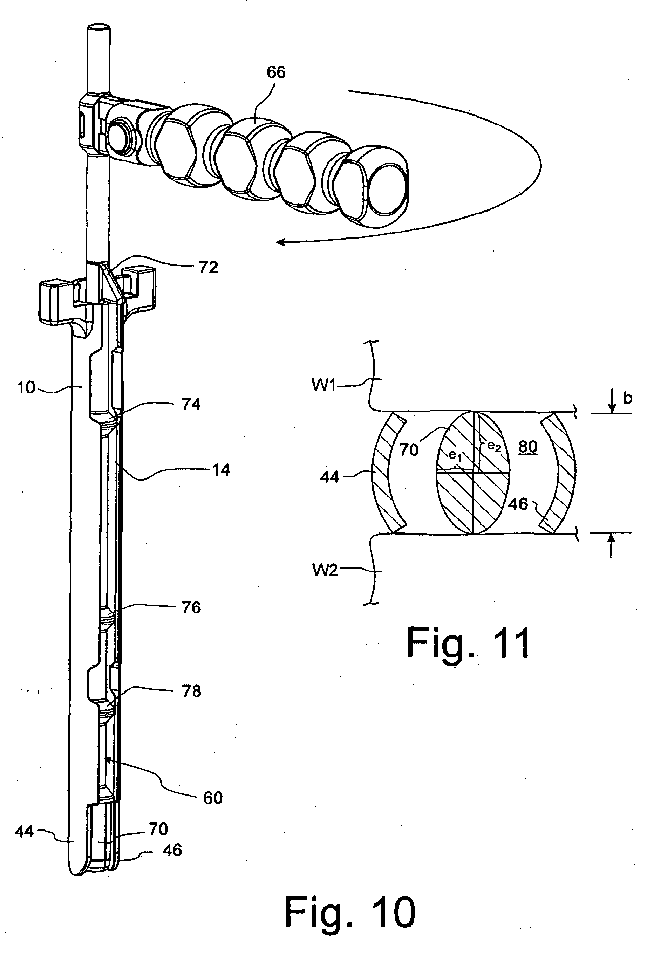

In einer bestimmten Axialposition berühren die Distanzzungen 44, 46 erstmals die Wirbel W1, W2. Die Hülse 10 wird nun noch ein Stück weiter abgesenkt; falls dazu größere Kräfte erforderlich sind, kann der Chirurg vorsichtig mit einem Hammer oder einem anderen Einschlaginstrument auf die Einwirkflächen 24, 26 am proximalen Ende der Hülse 10 schlagen. Dieser Einführvorgang wird so lange fortgesetzt, bis die Auflageschultern 48, 50 am distalen Ende der Hülse 10 an den Wirbelrändern der benachbarten Wirbel W1, W2 anschlagen. Möglich wird dieser Anschlag, weil der Außenabstand a zwischen der ersten Auflageschulter 48 einerseits und den zweiten Auflageschultern 50 andererseits entlang der Transversalrichtung größer ist als die Breite b der Distanzzungen 44, 46, die in das Bandscheibenfach 80 hineinreichen (vgl. Figur 3). Wenn diese Endposition erreicht ist, befinden sich die Distanzzungen 44, 46 der Hülse 10 auf der Höhe des Distraktionskopfes 70 des Distraktors 60, wie dies in der perspektivischen Darstellung der Figur 10 gezeigt ist.

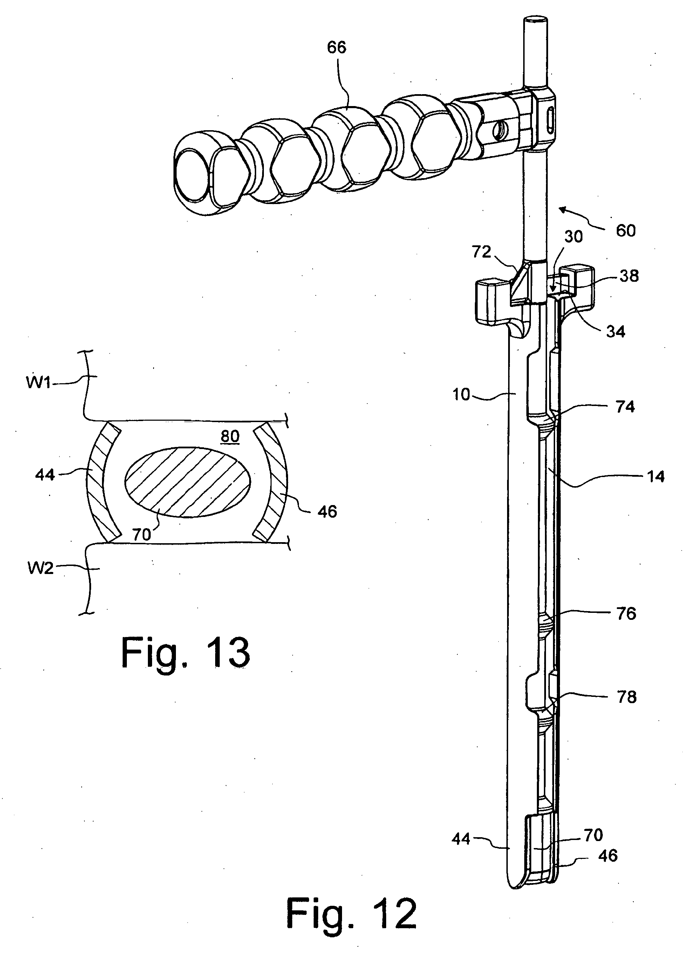

Die Distanzzungen 44, 46 nehmen in dieser Axialposition eine Stellung im Bandscheibenfach 80 ein, die in der Figur 11 gezeigt ist. Wie bereits erwähnt, entspricht die Breite b der Distanzzungen 44, 46 der Länge 2·e₂, während der Abstand a zwischen den Distanzzungen 44, 46 größer ist als 2·e₂. Auf diese Weise bleibt der Abstand zwischen den Wirbeln W1, W2 erhalten, wenn in einem sechsten Schritt der Distraktor 60 im Bandscheibenfach wieder um 90° gedreht wird, so dass der Distraktor 60 die in der Figur 12 gezeigte Stellung einnimmt. Während dieser Drehung um 90° wird die Nase 72 des Distraktors 60 in eine der Ausnehmungen 28, 30 in den Vorsprüngen 20, 22 der Hülse 10 gedreht und über eine der axialen Anschlagsflächen 32, 34 geführt. Somit kann es während der Drehbewegung nicht dazu kommen, dass der Distraktor 60 versehentlich noch tiefer in das Bandscheibenfach 80 eingeführt wird. Die Hülse 10 selbst kann nicht tiefer in das Bandscheibenfach 80 eindringen, da ihre Auflageschultern 48, 50 auf den Rändern der benachbarten Wirbel W1, W2 aufliegen.

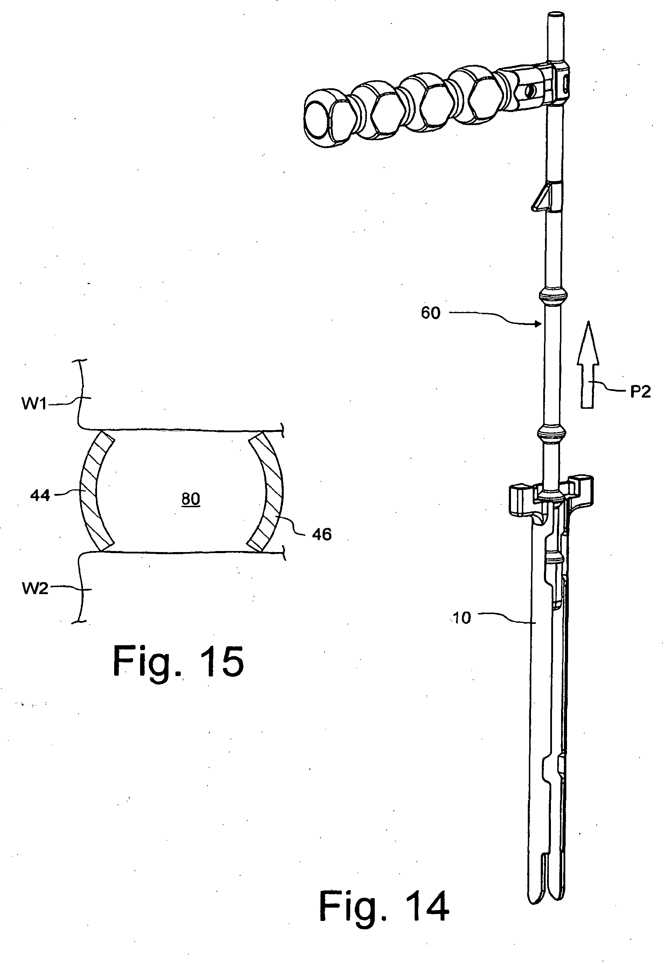

Nach dieser Drehung des Distraktors 60 um 90° stützen sich die Wirbel W1, W2 ausschließlich auf den Distanzzungen 44, 46 der Hülse 10 ab, wie dies im Querschnitt der Figur 13 erkennbar ist. Der Distraktionskopf 70 des Distraktors 60 ist somit vollständig entlastet, so dass der Distraktor 60 in einem siebten Schritt vollständig in dieser Drehposition aus der Hülse 10 herausgezogen werden kann, wie dies in der perspektivischen Darstellung der Figur 14 erkennbar und mit einem Pfeil P2 angedeutet ist. Ein seitliches Herausnehmen aus der Hülse 10 wird nicht ermöglicht, da sich die Hülse 10 bereits teilweise in dem engen Zugangskanal zum Bandscheibenfach 80 befindet. Dadurch ist über der Operationsstelle auch genügend Platz, um den Distraktor 60 einschließlich des Drehgriffs 66 herauszuziehen. Wie der Querschnitt der Figur 15 zeigt, befinden sich im Bandscheibenfach 80 nun lediglich die beiden Distanzzungen 44, 46 der Hülse 10.

Ein ggf. vorgesehenes Operationsmikroskop kann weggeschwenkt werden, da es für den nachfolgenden achten Schritt ohnehin im Allgemeinen nicht benötigt wird.

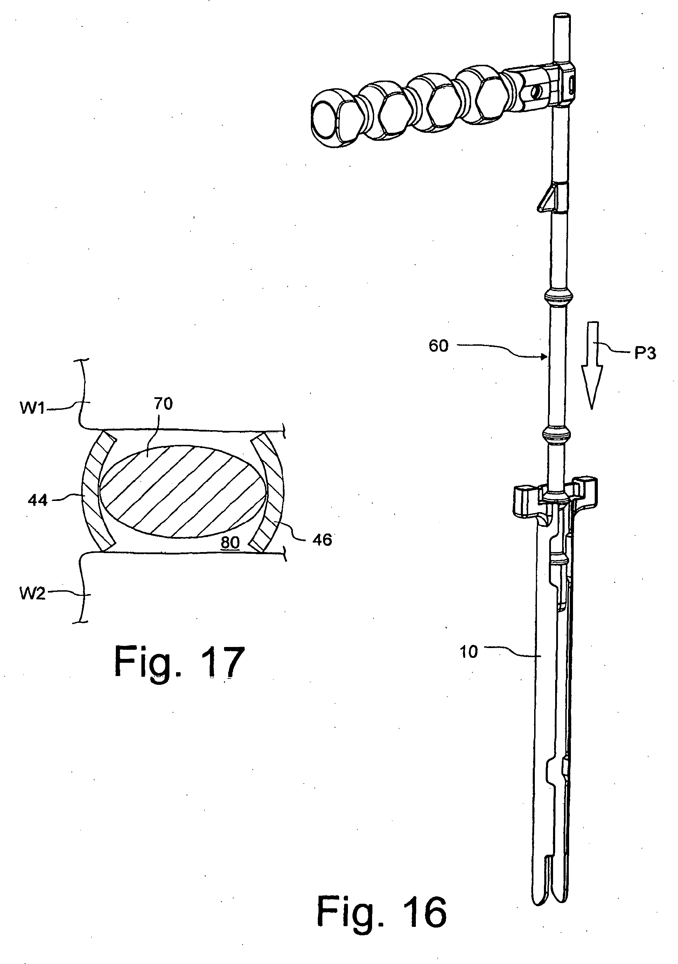

In diesem achten Schritt wird ein Distraktor 60 mit einem größeren Distraktionskopf 70 in die Hülse 10 eingeführt, wie in der Figur 16 durch einen Pfeil P3 angedeutet ist. Der Distraktionskopf 70 dieses anderen Distraktors 60 ist so dimensioniert, dass er in der Drehposition, wie er im Schnitt der Figur 17 gezeigt ist, noch zwischen den beiden Distanzzungen 44, 46 Platz findet. Die Abmessung 2·e₂ des Distraktionskopfs 70 ist somit geringfügig kleiner als der Abstand a zwischen den Distanzzungen 44, 46.

Am Ende des Einführvorgangs ragt der größere Distraktionskopf 70 in das Bandscheibenfach hinein, wie dies die Figur 17 zeigt. Die Nase 72 setzt schließlich auf eine der axialen Anschlagsflächen 32, 34 der Hülse 10 auf, wodurch ein zu tiefes Eindringen des Distraktionskopfes 70 in das Bandscheibenfach 80 verhindert wird. In dieser Drehstellung des Distraktors 60 liegt seine Nase 72 seitlich an einer der Drehanschlagsflächen 36, 38 am proximalen Ende der Hülse 10 an.

In einem neunten Schritt wird der größere Distraktor 60 in der Hülse 10 um 90° verdreht, wobei die Nase 72 zu Beginn der Drehbewegung noch auf einer der axialen Anschlagsflächen 32, 34 aufliegt. Die Drehanschlagsflächen 36, 38 verhindern dabei im Zusammenwirken mit der Nase 72, dass der Distraktor 60 in die falsche Richtung gedreht wird. Bei einer Drehung im falschen Drehsinn würde nämlich der Griff 66 nicht zum Längsschlitz 14, sondern in die entgegengesetzte Richtung (d. h. in der Figur 10 vom Betrachter weg) weisen, so dass die Hülse 10 nicht mehr seitlich vom Distraktor 60 abgezogen werden könnte.

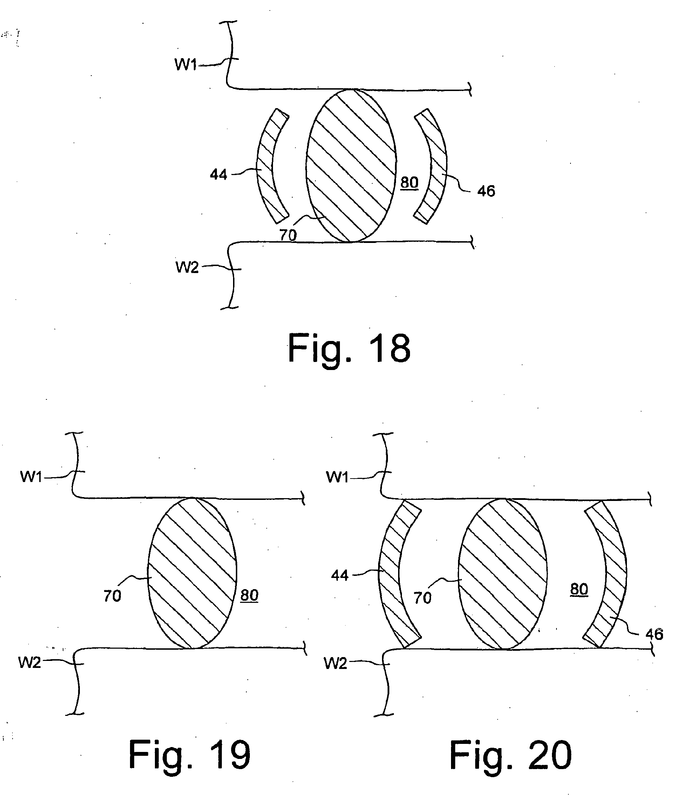

Der Distraktionskopf 70 richtet sich durch die Drehung des Distraktors 60 im Bandscheibenfach 80 auf und distrahiert dieses, wie dies im Querschnitt der Figur 18 gezeigt ist. Der Betrag der Distraktion beträgt auch hier wieder 2(e₂-e₁).

Durch die erneute Distraktion werden die beiden Distanzzungen 44, 46 der Hülse 10 wieder entlastet, so dass die Hülse 10 in einem zehnten Schritt aus dem Zugangskanal herausgezogen werden kann (Figur 19). Sobald die Hülse 10 eine Position erreicht, die der in der Figur 8 (allerdings für den vorher verwendeten Distraktor) gezeigten Position entspricht, kann die Hülse 10 seitlich von dem Distraktor 60 abgezogen werden.

In einem elften Schritt wird eine Hülse 10 mit dem nächstgrößeren Innendurchmesser ausgewählt, seitlich auf den Distraktor 60 aufgesetzt und dann axial abgesenkt, bis die Distanzzungen 44, 46 der größeren Hülse 10 in das Bandscheibenfach 80 hineinreichen (Figur 20). Die Breite b der Distanzzungen 44, 46 der jetzt verwendeten Hülse 10 ist dabei zumindest annähernd gleich der Höhe des bereits zweimal distrahierten Bandscheibenfachs 80 und beträgt somit 2·e₂, wobei e₂ die Länge der langen Halbachse des zuletzt verwendeten Distraktors 60 ist.

Der zuletzt eingeführte Distraktor 60 kann nun wieder in der oben bereits geschilderten Weise nach einer Drehung um 90° aus der Hülse 10 herausgezogen werden. Falls der Chirurg zu dem Ergebnis kommt, dass nach der Distraktion der Wirbel W1, W2 die Höhe des Bandscheibenfachs 80 für ein einzusetzendes Implantat ausreichend ist, so kann das Implantat durch die Hülse 10 hindurch in das Bandscheibenfach 80 eingeführt werden. Gegebenenfalls wird vorher noch mit Hilfe eines Bohrers der Zugangs an der dorsalen hinteren Wirbelkante aufgebohrt.

Kommt der Chirurg hingegen zu dem Ergebnis, dass eine weitere Distraktion erforderlich ist, so führt er in die Hülse 10 den Distraktor 60 mit dem nächstgrößeren Distraktionskopf ein und wiederholt die Schritte, die oben mit Bezug auf die Figuren 16 bis 20 beschrieben wurden. Dieser Distraktor 60 hat dann einen Distraktionskopf 70, bei dem für die Länge der langen Halbachse e₂ die Beziehung b < 2·e₂ < a gilt.

Diese Schritte können so lange wiederholt werden, bis das Bandscheibenfach 80 die gewünschte Höhe hat oder nicht mehr weiter distrahiert werden kann, ohne dass der die Wirbelsäule umgebende Bänder- und Muskelapparat des Patienten Schaden nimmt. Dies lässt sich für den Chirurgen durch visuelle Inspektion des Bänder- und Muskelapparats erkennen.

## Patentansprüche

1. System zum Distrahieren eines Bandscheibenfachs (80), mit:
a) mehreren Hülsen (10), wobei
- jede Hülse (10) an einem distalen Ende zwei Distanzzungen (44, 46) hat, die senkrecht zur Längsrichtung (12) der Hülse (10) eine Breite (b) haben, wobei
- sich die Hülsen (10) in der Breite (b) ihrer Distanzzungen (44, 46) voneinander unterscheiden, und mit
b) mehreren Distraktoren (60), wobei
- jeder Distraktor (60) ein zumindest im Wesentlichen stabförmiges Hauptteil (62) hat, an dessen distalem Ende ein Distraktionskopf (70) angeordnet ist, der im Querschnitt unterschiedliche Abmessungen in zwei orthogonalen Richtungen und insbesondere die Form eines Ovals hat, und wobei
- jeder Distraktor (60) in mindestens eine Hülse (10) einführbar und darin um seine Längsachse (68) verdrehbar ist,
**dadurch gekennzeichnet, dass**
jede Hülse (10) mit einem sich über ihre gesamte Länge erstreckenden Längsschlitz (14) versehen ist, so dass sie seitlich auf einen der Distraktoren (60) aufsetzbar ist.

2. System nach Anspruch 1, **dadurch gekennzeichnet, dass** an dem Hauptteil (68) der Distraktoren (60) ein Bereich (64, 74, 76, 78) mit vergrößertem Querschnitt vorgesehen ist, der zu einem fensterartig erweiterten Bereich (16, 18, 40, 42) im Längsschlitz (14) der Hülsen (10) korrespondiert, so dass jede Hülse (10) nur in einer einzigen axialen Relativposition seitlich auf einen der Distraktoren (60) aufsetzbar ist.

3. System nach Anspruch 2, **dadurch gekennzeichnet, dass** das Hauptteil (68) einen insgesamt kreisförmigen Querschnitt hat, und dass der Bereich mit vergrößertem Querschnitt durch eine ringförmige Verdickung (74, 76, 78) gebildet wird.

4. System nach einem der vorhergehenden Ansprüche, **dadurch gekennzeichnet, dass** das System einen Griff (66) aufweist, der lösbar an unterschiedlichen Distraktoren (60) befestigbar ist.

5. System nach Anspruch 2 und nach Anspruch 4, **dadurch gekennzeichnet, dass** der Bereich mit vergrößertem Querschnitt durch einen Befestigungsabschnitt (64) gebildet wird, der an den Distraktoren (60) zum lösbaren Befestigen des Griffs (66) ausgebildet ist.

6. System nach einem der vorhergehenden Ansprüche, **gekennzeichnet durch** Axialbegrenzer, welche die axiale Beweglichkeit der Distraktoren in den Hülsen in distaler Richtung zumindest in einer Drehstellung der Distraktoren begrenzen.

7. System nach Anspruch 6, **dadurch gekennzeichnet, dass** die Axialbegrenzer so ausgebildet sind, dass der Distraktionskopf (70) der Distraktoren (60) zumindest in einer Drehstellung der Distraktoren nicht über das distale Ende der jeweiligen Hülse (10) hinausgeführt werden kann.

8. System nach Anspruch 6 oder 7, **dadurch gekennzeichnet, dass** jeder Axialbegrenzer eine Nase (72), die seitlich vom stabförmigen Hauptteil (62) der Distraktoren (60) absteht, und eine an den Hülsen (10) ausgebildete und mit der Nase (72) zusammenwirkende axiale Anschlagsfläche (32, 34) umfasst.

9. System nach einem der vorhergehenden Ansprüche, **gekennzeichnet durch** Drehbegrenzer, welche die Beweglichkeit der Distraktoren (60) in den Hülsen (10) in Drehrichtung zumindest dann begrenzen, wenn sich die Distraktoren (60) in den jeweiligen Hülsen (10) in den **durch** die Axialbegrenzer vorgegebenen axialen Endpositionen befinden.

10. System nach Anspruch 8 und nach Anspruch 9, **dadurch gekennzeichnet, dass** jeder Drehbegrenzer die Nase (72) und eine mit der Nase (72) zusammenwirkende Drehanschlagsfläche (36, 38) umfasst.

11. System nach Anspruch 10, **dadurch gekennzeichnet, dass** die axiale Anschlagsfläche (32, 34) und die Drehanschlagsfläche (36, 38) durch Ausnehmungen (28, 30) definiert sind, die in zwei diametral von der Hülse (10) abgehenden Vorsprüngen (20, 22) gebildet ist.

12. System nach einem der vorhergehenden Ansprüche, **dadurch gekennzeichnet, dass** jede Hülse (10) am proximalen Ende eine radial vorspringende Einwirkfläche (24, 26) hat, sich in einer Ebene senkrecht zur Längsrichtung (12) der Hülse (10) erstreckt.

13. System nach Anspruch 12, **dadurch gekennzeichnet, dass** die Einwirkfläche (24, 26) an einem der Vorsprünge (20, 22) ausgebildet ist.

14. System nach einem der vorhergehenden Ansprüche, **dadurch gekennzeichnet, dass** die Distanzzungen (44, 46) der Hülsen (10) Teile einer Hülsenwand und durch zwei Aussparungen (40, 42) in der Hülsenwand definiert sind, die sich am distalen Ende der Hülsen einander gegenüberliegen.

15. System nach einem der vorhergehenden Ansprüche, **dadurch gekennzeichnet, dass** die Hülsen (10) zwischen den Distanzzungen (44, 46) Auflageschultern (48, 50) haben, deren Außenabstand (a) voneinander größer ist als die Breite der Distanzzungen (44, 46).

## Claims

1. System for distracting an intervertebral disc space (80), comprising
a) a plurality of sleeves (10), wherein
- each sleeve (10) has at a distal end two spacer tongues (44, 46) each having a width (b) perpendicular to the longitudinal direction (12) of the sleeve (10),
- the sleeves (10) differ from one another in the width (b) of their spacer tongues (44, 46), and
b) a plurality of distractors (60), wherein
- each distractor (60) has an at least substantially rod-shaped main part (62) having a distal end at which a distraction head (70) is arranged, which, in cross-section, has different dimensions in two orthogonal directions and, in particular, has the shape of an oval, and wherein
- each distractor (60) is able to be introduced into at least one sleeve (10) and turned therein about its longitudinal axis (68),
**characterized in that**
each sleeve (10) is provided with a longitudinal slot (14) extending over its entire length such that it can be placed laterally onto one of the distractors (60).

2. System according to Claim 1, **characterized in that** a region (64, 74, 76, 78) having an enlarged cross-section, which corresponds to a region (16, 18, 40, 42) in the longitudinal slot (14) of the sleeves (10) that is widened in the manner of a window, is provided on the main part (68) of the distractors (60) such that only in a single axial relative position each sleeve (10) can be placed laterally onto one of the distractors (60).

3. System according to Claim 2, **characterized in that** the main part (68) has an overall circular cross-section, and the region having an enlarged cross-section is constituted by an annular bulge (74, 76, 78).

4. System according to any one of the preceding claims, **characterized in that** the system has a handle (66) that can be detachably fastened to different distractors (60).

5. System according to Claim 2 and to Claim 4, **characterized in that** the region having an enlarged cross-section is constituted by a fastening portion (64) that is realized on the distractors (60) for the purpose of detachably fastening the handle (66).

6. System according to any one of the preceding claims, **characterized by** axial delimiters that delimit the axial mobility of the distractors in the sleeves in the distal direction, at least in one rotary position of the distractors.

7. System according to Claim 6, **characterized in that** the axial delimiters are realized such that at least in one rotary position of the distractors, the distraction head (70) of the distractors (60) cannot be moved beyond the distal end of the respective sleeve (10).

8. System according to Claim 6 or 7, **characterized in that** each axial delimiter comprises a lug (72), which projects laterally from the rod-shaped main part (62) of the distractors (60), and an axial stop surface (32, 34), which is realized on the sleeves (10) and acts together with the lug (72).

9. System according to any one of the preceding claims, **characterized by** rotary delimiters that delimit the mobility of the distractors (60) in the sleeves (10) in the rotary direction, at least when the distractors (60) in the respective sleeves (10) are in the axial end positions defined by the axial delimiters.

10. System according to Claim 8 and according to Claim 9, **characterized in that** each rotary delimiter comprises the lug (72) and a rotary stop surface (36, 38) that acts together with the lug (72).

11. System according to Claim 10, **characterized in that** the axial stop surface (32, 34) and the rotary stop surface (36, 38) are defined by recesses (28, 30) formed in two projections (20, 22) that project diametrically from the sleeve (10).

12. System according to any one of the preceding claims, **characterized in that** each sleeve (10), at the proximal end, has a radially projecting action surface (24, 26) that extends in a plane perpendicular to the longitudinal direction (12) of the sleeve (10).

13. System according to any one of the preceding claims, **characterized in that** the action surface (24, 26) is formed on one of the projections (20, 22).

14. System according to any one of the preceding claims, **characterized in that** the spacer tongues (44, 46) of the sleeves (10) are parts of a sleeve wall and defined by two cut-outs (40, 42) in the sleeve wall that are opposite one another at the distal end of the sleeves.

15. System according to any one of the preceding claims, **characterized in that** between the spacer tongues (44, 46) the sleeves (10) have bearing shoulders (48, 50) whose outer distance (a) from one another is greater than the width of the spacer tongues (44, 46).

## Revendications

1. Système de distraction d'un espace intervertébral (80),
comprenant :
a) plusieurs douilles (10), sachant que
- chaque douille (10) comporte, à une extrémité distale, deux languettes d'espacement (44, 46) présentant une largeur (b) perpendiculairement à la direction longitudinale (12) de ladite douille (10), sachant que
- les douilles (10) se différencient les unes des autres quant à la largeur (b) de leurs languettes d'espacement (44, 46), et
b) plusieurs distracteurs (60), sachant que
- chaque distracteur (60) comporte une partie principale (62) au moins pour l'essentiel en forme de tige, à l'extrémité distale de laquelle se trouve une tête de distraction (70) offrant des dimensionnements différents en coupe transversale, dans deux directions orthogonales, et revêtant notamment la forme d'un ovale, et sachant que
- chaque distracteur (60) peut être inséré dans au moins une douille (10) et peut tourner, dans cette dernière, autour de son axe longitudinal (68),
**caractérisé par le fait que**
chaque douille (10) est pourvue d'une fente longitudinale (14) s'étendant sur toute sa longueur, de telle sorte qu'elle puisse être mise en place latéralement sur l'un des distracteurs (60).

2. Système selon la revendication 1, **caractérisé par le fait qu'**une région (64, 74, 76, 78) à section transversale agrandie, prévue sur la partie principale (62) des distracteurs (60), concorde avec une région (16, 18, 40, 42) à élargissement du type fenêtre, dans la fente longitudinale (14) des douilles (10), de telle sorte que chaque douille (10) ne puisse être mise en place latéralement, sur l'un des distracteurs (60), que dans une unique position relative axiale.

3. Système selon la revendication 2, **caractérisé par le fait que** la partie principale (62) présente une section transversale globalement circulaire ; et **par le fait que** la région à section transversale agrandie est matérialisée par un renflement annulaire (74, 76, 78).

4. Système selon l'une des revendications précédentes, **caractérisé par le fait que** ledit système est muni d'une poignée (66) pouvant être fixée amoviblement à différents distracteurs (60).

5. Système selon la revendication 2 et selon la revendication 4, **caractérisé par le fait que** la région à section transversale agrandie est matérialisée par une zone de fixation (64) ménagée, sur les distracteurs (60), en vue de la fixation amovible de la poignée (66).

6. Système selon l'une des revendications précédentes, **caractérisé par** des limiteurs axiaux qui limitent la mobilité axiale des distracteurs dans les douilles, dans la direction distale, au moins dans une position que lesdits distracteurs ont prise par rotation.

7. Système selon la revendication 6, **caractérisé par le fait que** les limiteurs axiaux sont réalisés de façon telle que la tête de distraction (70) des distracteurs (60) ne puisse pas outrepasser l'extrémité distale de la douille (10) respective, au moins dans une position que lesdits distracteurs ont prise par rotation.

8. Système selon la revendication 6 ou 7, **caractérisé par le fait que** chaque limiteur axial inclut un bec (72) faisant saillie latéralement au-delà de la partie principale (62) des distracteurs (60), configurée en une tige, et une surface axiale (32, 34) de venue en butée qui est ménagée sur les douilles (10) et coopère avec ledit bec (72).

9. Système selon l'une des revendications précédentes, **caractérisé par** des limiteurs de rotation qui limitent la mobilité des distracteurs (60) dans les douilles (10), dans la direction de rotation, au moins lorsque lesdits distracteurs (60) occupent, dans les douilles (10) respectives, les emplacements extrêmes axiaux préétablis par les limiteurs axiaux.

10. Système selon la revendication 8 et selon la revendication 9, **caractérisé par le fait que** chaque limiteur de rotation inclut le bec (72) et une surface (36, 38) d'arrêt rotatoire, qui coopère avec ledit bec (72).

11. Système selon la revendication 10, **caractérisé par le fait que** la surface axiale (32, 34) de venue en butée, et la surface (36, 38) d'arrêt rotatoire, sont définies par des évidements (28, 30) pratiqués dans deux zones protubérantes (20, 22) dépassant de la douille (10) dans le sens diamétral.

12. Système selon l'une des revendications précédentes, **caractérisé par le fait que** chaque douille (10) comporte, à l'extrémité proximale, une surface opérante (24, 26) faisant saillie dans le sens radial et s'étendant dans un plan, perpendiculairement à la direction longitudinale (12) de ladite douille (10).

13. Système selon la revendication 12, **caractérisé par le fait que** la surface opérante (24, 26) est ménagée sur l'une des zones protubérantes (20, 22).

14. Système selon l'une des revendications précédentes, **caractérisé par le fait que** les languettes d'espacement (44, 46) des douilles (10) constituent des parties d'une paroi desdites douilles, et sont définies par deux dépouilles (40, 42) qui sont pratiquées dans ladite paroi des douilles et se trouvent en vis-à-vis mutuel à l'extrémité distale desdites douilles.

15. Système selon l'une des revendications précédentes, **caractérisé par le fait que** les douilles (10) comportent, entre les languettes d'espacement (44, 46), des épaulements d'appui (48, 50) mutuellement séparés d'une distance extérieure (a) excédant la largeur desdites languettes d'espacement (44, 46).
